# EUROPÄISCHE PATENTSCHRIFT

(11) **EP 2 467 022 B1**
(45) Veröffentlichungstag und Bekanntmachung des Hinweises auf die Patenterteilung: **15.08.2018**
(21) Anmeldenummer: 10754861.2
(22) Anmeldetag: 18.08.2010
(51) Int. Cl.: A01N 59/00

(54) **DESINFEKTIONSMITTEL, DESSEN VERWENDUNG UND DESINFEKTIONSVERFAHREN**
DISINFECTANT, USE THEREOF, AND DISINFECTION METHOD
PRODUIT DÉSINFECTANT, SON UTILISATION ET PROCÉDÉ DE DÉSINFECTION

(30) Priorität: 20.08.2009 DE 102009038213
(43) Veröffentlichungstag der Anmeldung: 27.06.2012
(73) Patentinhaber: Fresenius Medical Care Deutschland GmbH, 61352 Bad Homburg (DE)
(72) Erfinder: KIRCHNER, Ulrich, 61350 Bad Homburg (DE)
(74) Vertreter: Ackermann, Joachim
(86) Internationale Anmeldenummer: PCT/EP2010/005054
(87) Internationale Veröffentlichungsnummer: WO 2011/020597

(56) Entgegenhaltungen:
- WO-A1-00/42854
- WO-A1-03/044145
- CH-A- 506 295
- DE-A1- 1 767 992
- DE-A1-102005 006 104
- US-A- 4 675 120
- US-A- 5 019 288
- US-A- 5 202 047
- Henry E Wirth: "Apparent and Partial Molal Volumes of Sodium Chloride and Hydrochloric Acid in Mixed Solutions'", Journal of the American Chemical Society, vol. 62, no. 5, 1 May 1940 (1940-05-01), pages 1128-1134, XP055104914,

## Beschreibung

Die vorliegende Erfindung betrifft ein Desinfektionsmittel, dessen Verwendung zum Desinfizieren von medizinischen Geräten sowie ein Desinfektionsverfahren.

Das erfindungsgemäße Desinfektionsmittel kann als solches direkt zum Desinfizieren eingesetzt werden oder ergibt nach entsprechender Verdünnung ein Desinfektionsmittel, das gegenüber BaKterien und Viren, und insbesondere gegen extrem thermostabile Parvoviren einschließlich Hepatitis-Viren, wirksam ist und soll insbesondere die Desinfektion der Hydraulikteile von Dialysegeräten sowie von sonstigen medizinischen Geräten sowie Teilen derselben gewährleisten.

Säure enthaltende Reinigungsmittel sind im Stand der Technik bereits bekannt.

So offenbart DE 44 34 308 A1 ein Verfahren zum maschinellen Reinigen und Desinfizieren von Apparaten und Instrumenten, bei dem zunächst saure Reinigerflotten bei Temperaturen bis zu 55 °C zum Einsatz gelangen, sodann ein alkalischer Reiniger zudosiert, ein pH-Wert von mindestens 6,5 eingestellt und die Reinigung fortgesetzt wird, und anschließend mit Frischwasser nachgespült wird. Das Verfahren eignet sich zum Reinigen und Desinfizieren von medizinischen und biotechnologischen Apparaten und Instrumenten. Kombinationen von Säure und Salz werden nicht beschrieben.

DE 699 22 202 T2 offenbart ein Verfahren zur Beseitigung von Nanobakterien. Dabei werden kontaminierte Gegenstände einer ausgewählten desinfizierenden Mischung ausgesetzt, die Nanobakterien anschließend demineralisiert und der Gegenstand daraufhin mit Chemikalien desinfiziert oder Autoklaviert oder mit UV-Strahlung behandelt oder auf mindestens 60°C erhitzt. Das Demineralisieren kann durch Behandlung mit einer starken Säure erfolgen. Kombinationen von Säure und Salz werden nicht beschrieben.

DE 60 2004 006 490 T2 beschreibt ein Reinigungsmittel für harte Oberflächen, dass sich beispielsweise zur Reinigung von Badezimmern verwenden lässt. Neben einem Säurebestandteil umfasst dieses Reinigungsmittel neben Wasser mindestens zwei Tenside, von denen eines anionisch sein muss, suspendierte Einschlüsse auf der Basis von Alginaten und Verdickungsmittel. Derartige Reinigungsmittel eignen sich aufgrund ihrer zahlreichen biologisch abbaubaren Bestandteile nicht für die Reinigung von medizinischen Geräten. Kombinationen von Säure und Salz werden auch hier nicht beschrieben.

Aus der DE 600 01 914 T2 ist eine saure Zubereitung zum Inhibieren des mikrobiellen Wachstums bekannt, die sich für unterschiedliche Zwecke eignet, unter anderem als Reinigungs- und Desinfektionsmittel. Die Zubereitung umfasst mindestens zwei anorganische Säuren unterschiedlicher Stärke sowie mindestens eine Hydroxysäure und weist einen pH-Wert von kleiner als 1 auf. Kombinationen von Säure und Salz werden auch hier nicht beschrieben.

In der DE 1 767 992 A wird eine flüssige Reinigungsmischung offenbart, die sich zum Reinigen und Desinfizieren von porzellanartigen Installationen eignet. Die Mischung ist durch einen Gehalt an Netzmittel,an Säure und an pyrogenem Siliciumdioxid gekennzeichnet. Letzteres hat die Funktion eines Verdickers; die verdickte Flüssigkeit ist zum Aufbringen auf schwierig zu reinigende Flächen geeignet und zeichnet sich trotz der Gegenwart der Säure durch eine stabile Viskosität aus. In den Beispielen werden auch Varianten beschrieben, welche Kombinationen einer starken Säure mit Salzen aufweisen. Diese Mischung eignet sich aufgrund der Anwesenheit des Verdickungsmittels ebenfalls nicht für die Desinfektion von medizinischen Geräten.

Medizinische Hilfsmittel oder Geräte, die einem mehrfachen Gebrauch unterliegen, müssen in der Regel desinfiziert werden. Im Stand der Technik sind bereits unterschiedlichste Desinfektionsverfahren beschrieben worden.

So ist aus der DE 692 01 214 T2 ein Verfahren zur Entkeimung und Reinigung von Kontaktlinsen bekannt, bei dem ein gezielt instabilisiertes Wasserstoffperoxid zum Einsatz kommt. Dieses kann durch Einstellen eines pH-Wertes von mehr als 10 geschehen.

WO 00/42854 offenbart Zusammensetzungen zum Desinfizieren von medizinischen Geräten. Die aus dieser Schrift bekannten Zusammensetzungen basieren auf starken Säuren, wie z.B. auf Salzsäure, und besitzen bevorzugt einen pH-Wert von kleiner 1. Die zusätzliche Verwendung von Alkali- oder Erdalkalisalzen wird nicht offenbart. In diesem Dokument wird auf weitere saure Lösungen verwiesen, die in unterschiedlichsten Gebieten eingesetzt werden können.

Um die Infektion von Patienten durch kontaminierte medizinische Geräte, wie Dialysemaschinen, zu verhindern, werden die Geräte einer regelmäßigen Desinfektion unterzogen. Wichtig dabei ist es, die Inaktivierung aller potentiell vorhandenen Bakterien und Viren in möglichst kurzer Zeit bei möglichst niedrigen Temperaturen zu erreichen, um temperaturempfindliche Geräte bzw. Geräteteile zu schonen, mit minimalen Desinfektionszeiten auszukommen sowie den Strom- und Wärmeverbrauch minimal zu halten.

In der DIN EN 14476 werden Prüfverfahren für chemische Desinfektionsmittel und Antiseptika beschrieben. Dabei wird von einer viruziden Desinfektion gesprochen, wenn bei Temperaturen von bis zu 40°C die Virenlast von Adenoals auch von Polioviren um mindestens 4 log-Stufen reduziert wird. Die meisten Viren vertragen keine Temperaturen oberhalb von 40°C, so dass zur Prüfung einer allgemeinen Viruzidie hier besonders thermostabile Viren (Parvoviren), die bei diesen erhöhten Temperaturen auch chemostabil sind, Anwendung finden.

Da man bei der Desinfektion von medizinischen Geräten, beispielsweise im Wasserkreislauf einer Dialysemaschine, möglichst keine unverträglichen oder schwer zu entfernenden Substanzen verwenden möchte, werden bei der Desinfektion von medizinischen Geräten, wie Dialysemaschinen, chemothermische Desinfektionsverfahren angewendet, die möglichst wenig oder gar keine für den Patienten toxische Substanzen enthalten. Für diese wird in der DIN EN 14476 die Verwendung von Parvoviren zum Nachweis der desinfizierenden Wirkung vorgeschrieben.

Parvoviren sind Viren mit einsträngiger DNA. Diese besitzen keine Schutzhülle und zeichnen sich durch eine hohe Thermostabilität aus (Bräuniger et al., Zbl. Hyg. 196, S. 270-8 (1994)). Auch ihre verminderte Sensibilität gegenüber der viruziden Wirkung von organischen Säuren, wie Propionsäure, Zitronensäure und Essigsäure, wurde beschrieben (Werkerle 1988 Tierärztl. Umschau, 43, S. 654).

Genauer untersucht wurde die Inaktivierung der Parvoviren durch organische Säuren von Herbst et al. (Hyg. + Med., 15, (1990), S. 313-7). Hier wurde gezeigt, dass selbst bei 80°C nach 30 Minuten keine genügende Inaktivierung erreicht wird. Beim Einsatz von Ameisen- und Zitronensäure sind Temperaturen von 60-70°C und Inkubationszeiten von circa 30 Minuten notwendig, um einen ausreichenden Abfall des Virustiters zu erreichen.

In einer Veröffentlichung von Gerth (Hygiene und Infektionen im Krankenhaus, 1983, Gustav Fischer Verlag Frankfurt, ISBN 3 437-10815-8) wird die Widerstandsfähigkeit unterschiedlicher Viren unter bestimmten Einflüssen beschrieben. Dabei wird die Vermutung aufgestellt, dass bestimmte kleine DNA-Viren, wie Parvoviren, durch eine Langzeit-Pasteurisierung nicht inaktivert werden können.

EP 0 505 763 A1 beschreibt ein wässriges Desinfektionsmittel mit einem Gehalt an Zitronensäure als viruzidem Wirkstoff. In diesem Dokument wird eine Inaktivierung von Hepatitis-B-Viren bei Temperaturen von 20-75°C beschrieben.

Derzeit häufig eingesetzte Desinfektionsmittel für Dialysemaschinen verwenden Zitronensäure. Um eine Inaktivierung aller potenziell vorhandenen Viren zu gewährleisten, erfolgt die Desinfizierung der Dialysemaschinen derzeit bei 80°C.

Die Desinfektionsreinigung von Dialysemaschinen mit Zitronensäure enthaltenden Desinfektionsmitteln bei 85°C ist auch aus dem Handbuch "Grundlagen der Dialysetechnik" bekannt.

In der WO 95/10,349 A1 wird ein Verfahren zum Wiedereinsatz eines Dialysegerätes beschrieben. Dabei kommt eine 1-5 Gew. %-ige wässrige Zitronensäurelösung bei Temperaturen zwischen 90 und bis zu 100°C zum Einsatz, um pathogene Organismen und Viren abzutöten.

Aus einer Veröffentlichung von Poli et al. (G. Poli et al, Virucidal Activity of Organic Acids, Food Chemistry 1979, Seite 251-258) ist bekannt, dass organische Säuren "nackte" Viren bei den gewählten Konzentrationen nicht inaktivieren.

In der GB-A-2,103,089 wird ein Verfahren zur Unterbrechung oder Verhinderung der Ausbreitung von respiratorischen Viren beschrieben, bei dem Carbonsäuren, beispielsweise Citronensäure, Bernsteinsäure, Maleinsäure oder Salicylsäure, eingesetzt werden. Diese werden gegebenenfalls mit Tensiden kombiniert.

Eine Aufgabe der vorliegenden Erfindung besteht in der Bereitstellung eines Desinfektionsmittels, das - gegebenenfalls nach entsprechender Verdünnung - bei niedrigen Temperaturen, vorzugsweise zwischen 40 und 70°C, eine rasche und vollständige Desinfektion von medizinischen Geräten gestattet. Das erfindungsgemäße Desinfektionsmittelkonzentrat und das daraus hergestellte Desinfektionsmittel sind umweltfreundlich und hoch wirksam gegenüber Bakterien und Viren, die sich in Wasserkreisläufen sowie auf und/oder in medizinischen Geräten befinden. Das Desinfektionsmittel ist insbesondere wirksam gegenüber Parvoviren und somit gegenüber allen bekannten Virusarten und zeichnet sich durch hohe Wirksamkeit bei möglichst geringer Einwirkungszeit und möglichst niedrigen Einwirkungstemperaturen mit möglichst untoxischen bzw. bei der nachfolgenden Dialyse verwendeten Substanzen aus, so dass es sich insbesondere zur Desinfizierung von Wasserkreisläufen in Dialysemaschinen und von thermolabilen medizinischen Geräten sowie Teilen derselben, wie zum Beispiel von Dialysegeräten, in hohem Maße eignet.

Der Erfindung liegt die Aufgabe zugrunde, ein umweltfreundliches, untoxisches und gegenüber hochresistenten humanpathogenen Viren, insbesondere Hepatitis-B-Viren, und Bakterien, wirksames Desinfektionsmittel zur Verfügung zu stellen, das sich durch hohe Wirksamkeit bei möglichst geringer Einwirkungszeit und möglichst niedrigen Einwirkungstemperaturen auszeichnet, so dass es insbesondere zur Desinfizierung von thermolabilen medizinischen Geräten sowie Teilen derselben, wie zum Beispiel von Dialysegeräten, in hohem Maße geeignet ist.

Eine weitere Aufgabe der Erfindung besteht darin, der Desinfektion mit wässrigen Desinfektionsmitteln neue klinische Anwendungsbereiche zu erschließen, wobei auf dem neuen Verwendungsgebiet eine hohe Wirksamkeit bei möglichst geringer Einwirkungszeit und möglichst niedrigen Einwirkungstemperaturen sowie nichttoxischen Rückständen gewährleistet sein soll. Insbesondere in klinischen Bereichen, z.B. Dialyse, in denen die Verwendung von wässrigen Desinfektionsmitteln bislang nicht angezeigt war, und das Risiko einer Verschleppung von Desinfektionsmittelresten zu groß erschien, zeigt das erfindungsgemäße Desinfektionsmittel eine gute Wirksamkeit.

Eine weitere Aufgabe der vorliegenden Erfindung besteht in der Bereitstellung eines Desinfektionsverfahrens für Lumen und Flächen von medizinischen Geräten, das eine schnelle, schonende und auch umweltgerechte Desinfektion dieser Lumen und Flächen gestattet. Das erfindungsgemäße Desinfektionsverfahren zeichnet sich durch eine besonders hohe Wirksamkeit bei äußerst geringer Einwirkungszeit und bei äußerst niedrigen Einwirkungstemperaturen aus.

Die vorliegende Erfindung betrifft ein Desinfektionsmittel für medizinische Geräte oder Teile derselben, das auch als Konzentrat hergestellt werden kann, mit einem pH-Wert von kleiner gleich 1 enthaltend mindestens ein anorganisches Salz ausgewählt aus der Gruppe der Alkali- und/oder Erdalkalichloride und enhaltend mindestens Salzsäure als anorganische Säure zum Einstellen des pH-Wertes, die mit einer Carbonsäure kombiniert ist, und bei dem die Salzkonzentration mindestens 0,1 Gew. % beträgt.

Das erfindungsgemäße Desinfektionsmittel wird nachfolgend am Beispiel von Dialysemaschinen beschrieben. Es lässt sich aber auch für die Desinfektion anderer medizinischer Geräte einsetzen.

Unter medizinischen Geräten sind im Rahmen dieser Anmeldung derartige Vorrichtungen zu verstehen, welche der Verhütung, Überwachung, Behandlung oder Linderung von Krankheiten, oder der Erkennung, Überwachung, Behandlung, Linderung oder Kompensierung von Verletzungen oder Behinderungen oder der Untersuchung, Ersatz oder Veränderung des anatomischen Aufbaus oder eines physiologischen Vorgangs dienen. Beispiele für medizinische Geräte sind Infusionspumpen, Herz-Lungen-Maschinen, Dialysemaschinen oder Prothesen aller Art.

Das erfindungsgemäße Desinfektionsmittel zeichnet sich durch eine hohe antivirale Aktivität bereits bei niedrigen Temperaturen kombiniert mit kurzen Behandlungsdauern aus. So lassen sich beispielsweise bei Temperaturen von 30 bis 60°C und bei Behandlungsdauern von wenigen Minuten Parvoviren vollständig oder nahezu vollständig inaktivieren. Erreicht wird dieser Effekt durch die Verwendung einer Lösung mit hoher Salzkonzentration in Kombination mit einem niedrigen pH-Wert.

Das erfindungsgemäße Desinfektionsmittel kann als Feststoff bevorzugt jedoch als wässriges Desinfektionsmittel vorliegen.

Das erfindungsgemäße Desinfektionsmittel enthält als eine Komponente mindestens ein ausgewähltes Salz und eine ausgewählte Säure. Die Salzkonzentration im Desinfektionsmittel beträgt mindestens 0,1 Gew. %, vorzugsweise 0,3 Gew. % und insbesondere 0,4 Gew. % bis zur Sättigungsgrenze des Salzes.

Als Salz oder Säure wird eine dem Anwendungsgebiet angepasste Stoffauswahl eingesetzt. Im Falle der Desinfektion von Dialysegeräten kommen in der Regel solche Salze, Säuren oder Kombinationen von Salzen und/oder Säuren gemäß Anspruch 1 zum Einsatz, die auch beim bestimmungsgemäßen Gebrauch des Dialysegerätes eingesetzt werden.

Beispiele für besonders bevorzugt eingesetzte Alkali- und/oder Erdalkalichloride sind Natriumchlorid, Calciumchlorid, Magnesiumchlorid. Als anorganische Säure wird Salzsäure eingesetzt.

Gegebenenfalls kann das Desinfektionsmittel neben dem oder den Salzen bzw. Säuren noch einen Zucker, vorzugsweise Glucose, enthalten. In diesem Falle kann auch ein saures Dialysekonzentrat nach dem Zusetzen von Säure direkt als Desinfektionsmittel eingesetzt werden.

Gegebenenfalls kann das Desinfektionsmittel neben dem oder den Salzen bzw. Säuren und den gegebenenfalls anwesenden Zucker noch wasserlösliche Komplexbildner enthalten. Dabei handelt es sich in der Regel um wasserlösliche organische Substanzen mit chelatisierenden Eigenschaften. Ein Beispiel dafür ist Ethylendiamintetraessigsäure ("EDTA"). Auch Säuren, wie Zitronensäure, können komplexbildene Eigenschaften aufweisen; diese sind aber unter dem hier gewählten Begriff "Komplexbildner" nicht zu verstehen. Unter "wasserlöslich" ist im Rahmen dieser Beschreibung die Löslichkeit einer Substanz bei 25°C von mindestens 10 g/l Wasser zu verstehen.

Besonders bevorzugt besteht das erfindungsgemäße Desinfektionsmittel ausschließlich aus wasserlöslichen Substanzen und enthält diese in einer solchen Konzentration, dass das Desinfektionsmittel keine Feststoffe enthält. Alternativ dazu kann das Salz in einer solchen Konzentration vorliegen, dass das erfindungsgemäße Desinfektionsmittel einen festen Rückstand davon aufweist. Dieses trifft vor allem auf Desinfektionsmittel in der Form von Konzentraten zu. In der verdünnten Form enthält das erfindungsgemäße Desinfektionsmittel in der Regel nur gelöste Bestandteile.

Das erfindungsgemäße Desinfektionsmittel weist einen pH-Wert von kleiner gleich 1 auf, vorzugsweise von -1,5 bis +1 und ganz besonders bevorzugt von - 1,0 bis 0,5. Liegt das Desinfektionsmittel als Konzentrat vor, das vor der Anwendung verdünnt wird, so wird die Verdünnung so gewählt, dass sich im Desinfektionsmittel ein pH-Wert von kleiner gleich 1 ergibt.

Als Säure zum Einstellen des pH-Wertes wird ebenfalls eine dem Anwendungsgebiet angepasste Stoffauswahl eingesetzt. Im Falle der Desinfektion von Dialysegeräten kommt als anorganische Säure Salzsäure zum Einsatz. Diese wird mit Carbonsäuren, beispielsweise mit zwei- oder höherzähligen Säuren, wie Citronensäure, Oxalsäure, Malonsäure, Bernsteinsäure, Äpfelsäure, Glutarsäure, Hydroxyessigsäure oder Milchsäure und/oder mit anderen organischen Säuren, wie Essigsäure, Propionsäure oder Maleinsäure, kombiniert.

Das erfindungsgemäße Desinfektionsmittel kann - wie oben bereits dargelegt - ein oder mehrere organische Komplexbildner enthalten. Dabei kann es sich um komplexbildende organische Säuren und/oder sonstige organische Komplexbildner, wie EDTA, handeln.

Neben den oben dargelegten Bestandteilen kann das erfindungsgemäße Desinfektionsmittel weitere, für den vorgesehenen Anwendungszweck unschädliche Zusatzstoffe enthalten. Ein Beispiel dafür sind Farbstoffe.

Besonders bevorzugt enthält das erfindungsgemäße Desinfektionsmittel außer Säure, Salz, gegebenenfalls Wasser, gegebenenfalls Zucker und/oder gegebenenfalls Komplexbildner keine weiteren Zusätze.

Im Falle der Desinfektion von Dialysegeräten kommen vorzugsweise solche Säuren zum Einsatz, die auch beim bestimmungsgemäßen Gebrauch des Dialysegerätes eingesetzt werden. Beispiele dieser bevorzugten Säuren sind Salzsäure, insbesondere eine Kombination von Salzsäure mit Citronensäure und/oder mit Essigsäure und/oder mit Milchsäure.

Die Erfindung betrifft des Weiteren ein Desinfektionsmittel für medizinische Geräte oder Teilen davon, das durch Verdünnen eines Salz und Säure enthaltenden Desinfektionsmittelkonzentrats mit einer Salzkonzentration von mindestens 3 Gew. % mit Wasser erhältlich ist. Vorzugsweise wird das Desinfektionsmittel durch Verdünnen des Desinfektionsmittelkonzentrats auf 1-8 Gew. % erhalten.

Die Erfindung betrifft ferner ein Verfahren zum Desinfizieren von mit Bakterien, Pilzen und/oder Viren, insbesondere mit Hepatitis-B-Viren oder mit thermoresistenten Viren, wie Parvoviren, kontaminierten Lumen oder Flächen von medizinischen Geräten, insbesondere von Innenlumina und Innenflächen medizinischer Geräte sowie von Teilen derselben, wie z.B. Hydraulikteilen von Dialysemaschinen. Das Verfahren ist dadurch gekennzeichnet, dass man das oben beschriebene Desinfektionsmittel für eine solche Zeit bei einer Temperatur zwischen 30 und 70°C, vorzugsweise von 40 bis 60°C, auf die Lumen und/ oder Flächen von medizinischen Geräten oder Teilen davon einwirken lässt, bis mindestens 99,99 % der anfänglich vorhandenen Bakterien und/oder Viren inaktiviert sind. Dazu sind in der Regel Einwirkdauern zwischen 0,5 und 20 Minuten völlig ausreichend.

Da die desinfizierende Wirkung mit der Erhöhung der Temperatur und Einwirkzeit zunimmt, ist die untere Grenze des pH-Wertes und die obere Grenze der Salzkonzentration speziell kurzen Einwirkzeiten, beispielsweise zwischen 0,5 und 10 Minuten, und niedrigen Temperaturen, beispielsweise Temperaturen von 30-50°C zuzuordnen, während die oberen Grenzwerte für den pH und unteren Grenzwerten für die Salzkonzentration entsprechend höheren Temperaturen, beispielsweise Temperaturen von 40-70°C, sowie längeren Einwirkzeiten, beispielsweise 10 bis 20 Minuten, zuzuordnen.

Das erfindungsgemäße Desinfektionsmittel ermöglicht im Vergleich zu den bislang verwendeten, Citronensäure enthaltenden Desinfektionsmitteln, bei wesentlich geringeren Einwirkungszeiten und bei wesentlich geringeren Temperaturen eine vollständige Inaktivierung von Viren bzw. Abtötung von Bakterien. Außerdem zeichnet sich das erfindungsgemäße Desinfektionsmittel durch eine Umweltfreundlichkeit seiner viruziden Wirkstoffe aus.

Eine besondere Ausführungsform des erfindungsgemäßen Desinfektionsmittels liegt in der Form eines Konzentrats vor, das neben einer Säure zum Einstellen des pH-Wertes, wie Salzsäure, etwa 1-8 Gew. % Citronensäure als Klärungszusatz (Ca-Ionenfänger) enthält, und vorzugsweise in einer Konzentration von 1 Gew.-% bis zur Sättigungsgrenze, insbesondere von 2 bis 15 Gew.-%, und ganz besonders bevorzugt von 6 bis 10 Gew.-% Kochsalz.

Aus dem vorstehend beschriebenen Konzentrat werden vorzugsweise durch Verdünnen mit Wasser Desinfektionsmittel bereitet, welche 0,04 bis 0,32 Gew. % an Citronensäure in der Anwendungslösung als Klärungszusatz bzw. als Ca-Ionenfänger enthalten.

Der Einsatz eines Klärungszusatzes ist besonders vorteilhaft bei Geräten, in denen die zu desinfizierenden Flächen oder Lumen mit Verkalkungsrückständen wie CaCO₃ belastet sind. Diese Verkalkungsrückstände können die Wirksamkeit herkömmlicher Desinfektionsmittel vermindern. Das erfindungsgemäße Desinfektionsmittel zeigt aber auch bei einer CaCO₃ - Belastung eine gute Wirksamkeit wie in Testansatz 13 (s. Beispiele) gezeigt wird.

An Salzsäure zum Einstellen des pH-Werts werden vorzugsweise bis zu 8 Gew. % im Desinfektionsmittel eingesetzt.

Die Wirkung der Citronensäure kann durch Zugabe weiterer Säuren, insbesondere Äpfelsäure, Milchsäure und/oder Weinsäure verbessert werden. Zusätzlich können hierdurch Resistenzentwicklungen verhindert werden. Darüber hinaus kann die Neigung zur Kristallisation unterdrückt werden. Diese Säuren werden jeweils in einer Menge von 5 - 20 Gew-%, vorzugsweise etwa 10 Gew.-%, des Citronensäuregehalts eingesetzt.

Die erfindungsgemäße Lösung kann als Konzentrat bestehend im wesentlichen nur aus Säure und Salz(en) sowie gegebenenfalls darüber hinaus aus Wasser und/oder wasserlöslichem Chelatbildner bereitgestellt werden und entweder direkt oder in entsprechender Verdünnung mit Wasser eingesetzt werden. Falls erforderlich, kann diese Zusammensetzung am Ort des Einsatzes durch die Zugabe weiterer Säure auf den erforderlichen pH-Wert eingestellt werden.

Im Fall der Desinfektion von Dialysemaschinen kann dieses Konzentrat direkt mit einem dafür vorgesehenen Desinfektionsmittel-Anschluss an der Maschine angeschlossen werden. Die Verdünnung mit Wasser erfolgt dann automatisch in der Maschine.

Das erfindungsgemäße Desinfektionsmittel kann in einer alternativen Ausführungsform in Form einer anorganischen Säurelösung zur Verfügung gestellt werden, z.B. in Form einer Salzsäurelösung, die mit einer konzentrierten Salzlösung verdünnt wird. Ein Beispiel für eine solche Salzlösung kann das in der Dialyse überlicherweise verwendete saure Dialysekonzentrat sein. Es handelt sich um eine konzentrierte Elektrolytlösung, aus der z.B in der Dialysemaschine durch Verdünnung mit Wasser und Kombination mit einem Puffer die physiologischen Dialyselösungen hergestellt werden.

Diese anorganische Säurelösung wird am Ort des Einsatzes vorzugsweise mit dem üblicherweise bei der Dialyse verwendeten sauren Dialysekonzentrat auf die erforderliche Konzentration verdünnt und kann so direkt über den Dialysatzugang an der Maschine eingesetzt werden.

Ein weiterer Gegenstand der Erfindung ist die Verwendung des oben beschriebenen Desinfektionsmittels zur Inaktivierung von Bakterien und/oder Viren, insbesondere von Parvoviren, bei der Desinfektion von Lumen und/oder Flächen von medizinischen Geräten oder Teilen davon, insbesondere von thermolabilen medizinischen Geräten oder Teilen davon, ganz besonders bei der Desinfektion von Hydraulikkomponenten von Dialysegeräten.

Diese Verwendungen sind ebenfalls Gegenstand der vorliegenden Erfindung.

Die folgenden Beispiele dienen zur näheren Erläuterung der Erfindung. Soweit nicht anders angegeben, beziehen sich Prozentangaben auf das Gewicht:
Testansatz 1: 0,44% Zitronensäure + 0,1% HCl in 100%igem saurem Dialysekonzentrat bei 50°C; das Konzentrat ist innerhalb von 2 Min. wirksam (= Reduktionsfaktor Parvovirus >4,5)

| Temperatur | Belastung | Reduktionsfaktor (Ig) Parvovirus | | | |
|---|---|---|---|---|---|
| | | 1 Min. | 2 Min. | 5 Min. | 10 Min. |
| 50°C | Clean | >3,5 | >4,5 | >4,5 | >4,5 |

Testansatz 2: 0,1% HCl in 100%igem saurem Dialysekonzentrat bei 50°C; das Konzentrat ist innerhalb von 5 Min. wirksam (= Reduktionsfaktor Parvovirus >4,1)

| Temperatur | Belastung | Reduktionsfaktor (Ig) Parvovirus | | | |
|---|---|---|---|---|---|
| | | 1 Min. | 2 Min. | 5 Min. | 10 Min. |
| 50°C | clean | 2,5 | 3,8 | >4,1 | >4,1 |

Testansatz 3: 0,44% Zitronensäure + 0,03% HCl in 100%igem saurem Dialysekonzentrat bei 50°C; C; das Konzentrat ist innerhalb von 10 Min. wirksam (= Reduktionsfaktor Parvovirus >4,25)

| Temperatur | Belastung | Reduktionsfaktor (Ig) Parvovirus | | | |
|---|---|---|---|---|---|
| | | 1 Min. | 2 Min. | 5 Min. | 10 Min. |
| 50°C | clean | 1,75 | 2,63 | >3,25 | >4,25 |

Testansatz 4: 0,48% Zitronensäure + 0,1% HCl in 100%igem saurem Dialysekonzentrat (saures Dialysekonzentrat = 270,87 g NaCl + 8,27 g CaCl₂ x 2H₂O + 8,11 g Essigsäure + 6,71 g KCl + 4,57 g MgCl₂x6H₂O + 49,5 g Glucose ad 1000 mL; pH: 2,0); C; das Konzentrat ist innerhalb von 5 Min. bei 42°C wirksam (= Reduktionsfaktor Parvovirus >4,25)

| Temperatur | Belastung | Reduktionsfaktor (Ig) Parvovirus | | | |
|---|---|---|---|---|---|
| | | 1 Min. | 2 Min. | 5 Min. | 10 Min. |
| 42°C | clean | 0,87 | 2,87 | >4,25 | >4,25 |

Testansatz 5: 50%iges saures Dialysekonzentrat + 0,12% HCl; C; das Konzentrat ist innerhalb von 15 Min. bei 50°C wirksam (= Reduktionsfaktor Parvovirus >4,37)

| Temperatur | Belastung | Reduktionsfaktor (Ig) Parvovirus | | | |
|---|---|---|---|---|---|
| | | 2 Min. | 5 Min. | 10 Min. | 15 Min. |
| 50°C | clean | 0,87 | 2,62 | 3,25 | >4,37 |

Testansatz 6: 100%iges saures Dialysekonzentrat bei 50°C; C; das Konzentrat ist innerhalb von 10 Min. bei 50°C unwirksam (= Reduktionsfaktor Parvovirus 1,12)

| Temperatur | Belastung | Reduktionsfaktor (Ig) Parvovirus | | | |
|---|---|---|---|---|---|
| | | 1 Min. | 2 Min. | 5 Min. | 10 Min. |
| 50°C | clean | 0,12 | 0,00 | 0,50 | 1,12 |

Ab einer Salzkonzentration, die in etwa der von saurem Dialysekonzentrat entspricht (saures Dialysekonzentrat = 270,87 g NaCl + 8,27 g CaCl₂ x 2H₂O + 8,11 g Essigsäure + 6,71 g KCl + 4,57 g MgCl₂x6H₂O + 49,5 g Glucose ad 1000 mL; pH: 2,0) und ab einem Säurezusatz von 0,1% werden bei Temperaturen ab 42°C Parvoviren in 2 bis 10 Minuten um mehr als 4 log Stufen (99,99%) reduziert. Gleiche Salzkonzentrationen wirken bei pH - Werten >2,0 und gleichen Temperaturen demgegenüber nicht. Beim Erhöhen der Säurekonzentration wird die Wirkung von 10 auf 2 Minuten beschleunigt. Auch 50%iges saures Dialysekonzentrat genügt bei 50°C und Ansäuerung auf pH 0 für eine Wirksamkeit in 15 Minuten.
Testansatz 7: 10% NaCl + 0,12% HCl; das Konzentrat ist innerhalb von 15 Min. bei 50°C unwirksam (= Reduktionsfaktor Parvovirus 3,5)

| Temperatur | Belastung | Reduktionsfaktor (Ig) Parvovirus | | | |
|---|---|---|---|---|---|
| | | 2 Min. | 5 Min. | 10 Min. | 15 Min. |
| 50°C | clean | 1,00 | 2,12 | 3,12 | 3,50 |

Testansatz 8: 0,5% NaCl + 0,3% HCl; das Konzentrat ist innerhalb von 5 Min. bei 50°C wirksam (= Reduktionsfaktor Parvovirus >4,4)

| Temperatur | Belastung | Reduktionsfaktor (Ig) Parvovirus | | | |
|---|---|---|---|---|---|
| | | 2 Min. | 5 Min. | 10 Min. | 15 Min. |
| 50°C | clean | 2,90 | >4,4 | >4,5 | n.d. |

Die Konzentration an Kochsalz kann stark abgesenkt werden, wenn gleichzeitig die Säurekonzentration erhöht wird.
Testansatz 9: 0,48% NaCl + 0,08% Zitronensäure + 0,32% HCl; das Konzentrat ist innerhalb von 2,5 Min. bei 50°C wirksam (= Reduktionsfaktor Parvovirus >4,1)

| Temperatur | Belastung | Reduktionsfaktor (Ig) Parvovirus | | | |
|---|---|---|---|---|---|
| | | 2,5 Min. | 5 Min. | 10 Min. | 15 Min. |
| 50°C | clean | >4,1 | >4,4 | >4,4 | >4,4 |

Testansatz 10: 0,1% NaCl + 0,32% HCl; das Konzentrat ist innerhalb von 5 Min. bei 50°C wirksam (= Reduktionsfaktor Parvovirus >4,4)

| Temperatur | Belastung | Reduktionsfaktor (Ig) Parvovirus | | | |
|---|---|---|---|---|---|
| | | 2,5 Min. | 5 Min. | 10 Min. | 15 Min. |
| 50°C | clean | 2,50 | >4,4 | >4,4 | >4,4 |

Testansatz 11: 0,32% HCl bei 50°C; das Konzentrat ist innerhalb von 10 Min. bei 50°C wirksam (= Reduktionsfaktor Parvovirus >4,3)

| Temperatur | Belastung | Reduktionsfaktor (Ig) Parvovirus | | | |
|---|---|---|---|---|---|
| | | 2,5 Min. | 5 Min. | 10 Min. | 15 Min. |
| 50°C | clean | 2,60 | 3,4 | >4,3 | >4,3 |

Testansatz 12: 0,32% HCl; das Konzentrat ist bei Carbonatbelastung und innerhalb von 15 Min. bei 60°C unwirksam (= Reduktionsfaktor Parvovirus 0,9)

| Temperatur | Belastung | Reduktionsfaktor (Ig) Parvovirus | | | |
|---|---|---|---|---|---|
| | | 2,5 Min. | 5 Min. | 10 Min. | 15 Min. |
| 60°C | CaCO₃ | 0,5 | 0,5 | 0,25 | 0,9 |

Testansatz 13: 0,48% NaCl + 0,08% Zitronensäure + 0,32% HCl; das Konzentrat ist auch bei Carbonatbelastung innerhalb von 5 Min. bei 50°C wirksam (= Reduktionsfaktor Parvovirus 4,0)

| Temperatur | Belastung | Reduktionsfaktor (Ig) Parvovirus | | | |
|---|---|---|---|---|---|
| | | 2,5 Min. | 5 Min. | 10 Min. | 15 Min. |
| 50°C | clean | 3,9 | 4,0 | >4,0 | >4,0 |

Mit anorganischen Säuren versetzte Zitronensäure und saure Salzlösungen sind wirksam.

Auch Salzkonzentrationen von nur 0,48% und auch noch 0,1% sind gut wirksam, wenn die Säurekonzentration auf über 0,3% HCl erhöht wird.

Salzsäure alleine weist nicht diese Wirksamkeit auf und wird - im Gegensatz zur wirksamen Formulierung - mit Kalk schnell inaktiviert.

Unabdingbar für die Erfüllung der durch die Norm geforderten Bedingungen und die Verbesserung der zur Zeit verwendeten Desinkfektionsmittel ist also die Kombination beider Merkmale.

## Patentansprüche

1. Desinfektionsmittel für medizinische Geräte oder Teile davon mit einem pH-Wert von kleiner gleich 1 enthaltend mindestens ein anorganisches Salz ausgewählt aus der Gruppe der Alkali- und/oder Erdalkalichloride, und enthaltend mindestens Salzsäure als anorganische Säure zum Einstellen des pH-Wertes, die mit einer Carbonsäure kombiniert ist, und bei dem die Salzkonzentration mindestens 0,1 Gew. % beträgt.

2. Desinfektionsmittel gemäß Anspruch 1, **dadurch gekennzeichnet, dass** es sich um ein wässriges Desinfektionsmittel handelt.

3. Desinfektionsmittel gemäß Anspruch 1, **dadurch gekennzeichnet, dass** das Salz ausgewählt wird aus der Gruppe Natriumchlorid, Calciumchlorid oder Magnesiumchlorid, oder aus Kombinationen von zwei oder mehreren davon.

4. Desinfektionsmittel gemäß Anspruch 1, **dadurch gekennzeichnet, dass** dessen pH-Wert unter 0,5 beträgt.

5. Desinfektionsmittel gemäß Anspruch 1, **dadurch gekennzeichnet, dass** dessen pH-Wert 0,4 bis -1,5 beträgt.

6. Desinfektionsmittel gemäß Anspruch 1, **dadurch gekennzeichnet, dass** die anorganische Säure mit einer Carbonsäure ausgewählt aus der Gruppe Citronensäure, Oxalsäure, Malonsäure, Bernsteinsäure, Äpfelsäure, Glutarsäure, Hydroxyessigsäure, Milchsäure, Essigsäure, Propionsäure und/oder Maleinsäure kombiniert ist.

7. Desinfektionsmittel gemäß Anspruch 1, **dadurch gekennzeichnet, dass** die Säure zum Einstellen des pH-Wertes eine Kombination von Salzsäure mit Citronensäure und/oder mit Essigsäure und/oder mit Milchsäure ist.

8. Desinfektionsmittel gemäß Anspruch 1, **dadurch gekennzeichnet, dass** dieses durch Verdünnen eines Desinfektionsmittelkonzentrats mit einer Salzkonzentration von mindestens 3 Gew. % mit Wasser erhalten wird.

9. Desinfektionsmittel nach Anspruch 8, **dadurch gekennzeichnet, dass** das Desinfektionsmittel durch Verdünnen des Desinfektionsmittelkonzentrats auf 1-8 Gew. % erhältlich ist.

10. Desinfektionsmittel nach Anspruch 1, **dadurch gekennzeichnet, dass** dieses durch Kombination von Salzsäure als anorganische Säure mit einem sauren Dialysekonzentrat erhältlich ist, das bei der Dialyse eingesetzt wird.

11. Verfahren zum Desinfizieren von mit Pilzen und/oder Viren und/oder Bakterien kontaminierten Lumen und/oder Flächen medizinischer Geräte, **dadurch gekennzeichnet, dass** man ein Desinfektionsmittel nach Anspruch 1 bei einer Temperatur zwischen 30 und 70°C für 0,5 bis 20 Minuten auf die zu desinfizierenden Lumen und/oder Flächen einwirken lässt.

12. Verfahren gemäß Anspruch 11, **dadurch gekennzeichnet, daß** man das Desinfektionsmittel bei 40 bis 60°C mit den Lumen und/oder Flächen in Berührung bringt.

13. Verfahren gemäß Anspruch 11, **dadurch gekennzeichnet, dass** es sich bei den kontaminierten Lumen und/oder Flächen um die wasserführenden Teile von Dialysegeräten handelt.

14. Verwendung eines Desinfektionsmittels nach einem der Ansprüche 1 bis 10 zur Inaktivierung von Pilzen und/oder Viren und/oder Bakterien bei der Desinfektion von Lumen und/oder Flächen von medizinischen Geräten oder Teilen davon.

15. Verwendung nach Anspruch 14, **dadurch gekennzeichnet, dass** es sich bei dem medizinischen Gerät um ein Dialysegerät oder um ein Teil davon handelt.

## Claims

1. Disinfectant for medical devices or parts thereof having a pH-value of less than or equal to 1 comprising at least one inorganic salt selected from the group of alkaline and/or earth alkaline chlorides, and containing at least hydrochloric acid as inorganic acid to adjust the pH-value, which is combined with a carboxylic acid, and which has a salt concentration of at least 0.1 % by weight.

2. Disinfectant according to claim 1, **characterized in that** it is an aqueous disinfectant.

3. Disinfectant according to claim 1, **characterized in that** the salt is selected from the group of sodium chloride, calcium chloride or magnesium chloride, or from combinations of two or more of them.

4. Disinfectant according to claim 1, **characterized in that** its pH-value is less than 0.5.

5. Disinfectant according to claim 1, **characterized in that** its pH-value is 0.4 to -1.5.

6. Disinfectant according to claim 1, **characterized in that** the inorganic acid is combined with a carboxylic acid selected from the group of citric acid, oxalic acid, malonic acid, succinic acid, malic acid, glutaric acid, hydroxyacetic acid, lactic acid, acetic acid, propionic acid and/or maleic acid.

7. Disinfectant according to claim 1, **characterized in that** the acid for adjusting the pH-value is a combination of hydrochloric acid with citric acid and/or with acetic acid and/or with lactic acid.

8. Disinfectant according to claim 1, **characterized in that** this is obtained by diluting with water a disinfectant concentrate having a salt concentration of at least 3 % by weight.

9. Disinfectant according to claim 8, **characterized in that** the disinfectant is obtained by diluting the disinfectant concentrate to 1-8 % by weight.

10. Disinfectant according to claim 1, **characterized in that** this is obtainable by combining hydrochloric acid as an inorganic acid with an acidic dialysis concentrate, which is used in dialysis.

11. A method for disinfecting lumens and/or surfaces of medical devices contaminated with fungi and/or viruses and/or bacteria, **characterized in that** a disinfectant according to claim 1 interacts at a temperature between 30 and 70 °C for 0.5 to 20 minutes with the lumens and/or surfaces to be disinfected.

12. The method according to claim 11, **characterized in that** the disinfectant is in contact with the lumens and/or surfaces at 40 to 60 °C.

13. The method according to claim 11, **characterized in that** the contaminated lumens and/or surfaces are the water-carrying parts of dialysis devices.

14. Use of a disinfectant in accordance with one of the claims 1 to 10 for the inactivation of fungi and/or viruses and/or bacteria in the disinfection of lumens and/or surfaces of medical devices or parts thereof.

15. Use according to claim 14, **characterized in that** the medical device is a dialysis device or a part thereof.

## Revendications

1. Agent ou produit désinfectant pour appareils médicaux ou des parties de ceux-ci ayant un pH inférieur ou égal à 1, contenant au moins un sel inorganique choisi dans le groupe des chlorures alcalins et/ou alcalino-terreux, et contenant au moins de l'acide chlorhydrique en tant qu'acide inorganique pour l'ajustement du pH, qui est combiné avec un acide carboxylique, et dans lequel la concentration en sels est d'au moins 0,1 % en poids.

2. Agent désinfectant selon la revendication 1, **caractérisé en ce qu'**il s'agit d'un agent désinfectant aqueux.

3. Agent désinfectant selon la revendication 1, **caractérisé en ce que** le sel est choisi dans le groupe constitué par le chlorure de sodium, le chlorure de calcium ou le chlorure de magnésium, ou parmi des combinaisons de deux ou plus d'entre eux.

4. Agent désinfectant selon la revendication 1, **caractérisé en ce que** son pH est inférieur à 0,5.

5. Agent désinfectant selon la revendication 1, **caractérisé en ce que** son pH est de 0,4 à -1,5.

6. Agent désinfectant selon la revendication 1, **caractérisé en ce que** l'acide inorganique est combiné avec un acide carboxylique choisi dans le groupe constitué par l'acide citrique, l'acide oxalique, l'acide malonique, l'acide succinique, l'acide malique, l'acide glutarique, l'acide hydroxyacétique, l'acide lactique, l'acide acétique, l'acide propionique et/ou l'acide maléique.

7. Agent désinfectant selon la revendication 1, **caractérisé en ce que** l'acide pour l'ajustement du pH est une combinaison d'acide chlorhydrique avec de l'acide citrique et/ou avec de l'acide acétique et/ou avec de l'acide lactique.

8. Agent désinfectant selon la revendication 1, **caractérisé en ce que** celui-ci est obtenu par dilution d'un agent désinfectant concentré ayant une concentration en sels d'au moins 3 % en poids avec de l'eau.

9. Agent désinfectant selon la revendication 8, **caractérisé en ce que** l'agent désinfectant peut être obtenu par dilution de l'agent désinfectant concentré à 1 à 8 % en poids.

10. Agent désinfectant selon la revendication 1, **caractérisé en ce que** celui-ci peut être obtenu par combinaison d'acide chlorhydrique en tant qu'acide inorganique avec un concentré de dialyse acide qui est utilisé lors de la dialyse.

11. Procédé de désinfection de lumières et/ou de surfaces d'appareils médicaux contaminées avec des champignons et/ou des virus et/ou des bactéries, **caractérisé en ce qu'**un agent désinfectant selon la revendication 1 est laissé agir à une température comprise entre 30 et 70 °C pendant 0,5 à 20 minutes sur les lumières et/ou les surfaces à désinfecter.

12. Procédé selon la revendication 11, **caractérisé en ce que** l'agent désinfectant est mis en contact avec les lumières et/ou les surfaces à une température de 40 à 60 °C.

13. Procédé selon la revendication 11, **caractérisé en ce que** les lumières et/ou les surfaces contaminées sont les parties conduisant l'eau d'appareils de dialyse.

14. Utilisation d'un agent désinfectant selon l'une quelconque des revendications 1 à 10 pour l'inactivation de champignons et/ou de virus et/ou de bactéries lors de la désinfection de lumières et/ou de surfaces d'appareils médicaux ou de parties de ceux-ci.

15. Utilisation selon la revendication 14, **caractérisée en ce que** l'appareil médical est un appareil de dialyse ou une partie de celui-ci.
